(19) Europäisches Patentamt
European Patent Office
Office européen des brevets

(11) EP 2 342 210 B1

(12) EUROPEAN PATENT SPECIFICATION

(45) Date of publication and mention of the grant of the patent:
**23.07.2014 Bulletin 2014/30**

(21) Application number: **09789277.2**

(22) Date of filing: **08.09.2009**

(51) Int Cl.:
***C07F 9/50*** (2006.01) ***C07F 9/6574*** (2006.01)
***C07B 41/06*** (2006.01) ***C07C 45/50*** (2006.01)

(86) International application number:
**PCT/US2009/005046**

(87) International publication number:
**WO 2010/030339 (18.03.2010 Gazette 2010/11)**

# (54) ACETYLENE TOLERANT HYDROFORMYLATION CATALYSTS

ACETYLENTOLERANTE HYDROFORMYLIERUNGSKATALYSATOREN

CATALYSEURS D HYDROFORMYLATION TOLÉRANT L ACÉTYLÈNE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.09.2008 US 209626**

(43) Date of publication of application:
**13.07.2011 Bulletin 2011/28**

(73) Proprietor: **Eastman Chemical Company**
**Kingsport, TN 37660 (US)**

(72) Inventor: **PUCKETTE, Thomas, Allen**
**Longview**
**TX 75605 (US)**

(74) Representative: **Ricker, Mathias et al**
**Wallinger Ricker Schlotter Tostmann**
**Patent- und Rechtsanwälte**
**Zweibrückenstrasse 5-7**
**80331 München (DE)**

(56) References cited:
**WO-A1-99/13984**
**WO-A2-87/07600**
**WO-A2-2005/042458**
**US-A- 5 675 041**
**US-A- 6 049 011**
**US-A1- 2007 219 399**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

BACKGROUND OF THE INVENTION

The hydroformylation of ethylene to prepare propionaldehyde is a well known and important industrial reaction. The preparation of propionaldehyde is accomplished by the reaction of ethylene with hydrogen and carbon monoxide in the presence of a rhodium containing catalyst under conditions of elevated temperature and pressure. Ethylene from commercial sources such as refineries and cracking plants frequently contains acetylene in varying amounts. Typically, the acetylene is removed from the ethylene stream by partial hydrogenation. However, during times of upset operation, the ethylene product may be contaminated with varying levels of acetylene which reduces the catalyst activity in a non-permanent manner.

The effects of acetylene impurities on the hydroformylation of olefins, such as ethylene, vary with the nature of the trivalent phosphorus ligand that is used to stabilize the rhodium catalyst. Strongly basic phosphine ligands are more susceptible to the reversible poisoning effects of acetylene. When the acetylene contamination is in the single digit part per million level, the negative effects of acetylene are small. However, when the acetylene levels exceed single digit numbers, the loss of activity can be catastrophic to the reaction. Therefore, it is highly desirable to have a catalyst system that can withstand the acetylene impurity spikes without loss of catalyst activity.

WO 87/07600 A2 relates to hydroformylation reactions wherein at least one olefin having from 2 to 20 carbon atoms is contacted in a reaction zone at a temperature of from about 20 °C to about 250 °C and a pressure of from about 15 psig to about 800 psig with hydrogen, carbon monoxide, and a catalyst containing rhodium, at least one ligand having formula (I),

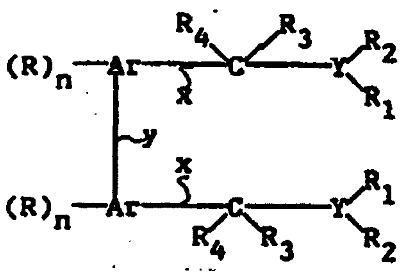

(I)

wherein each Ar is independently selected from aromatic ring compounds having 6 up to 14 carbon atoms, e.g., phenyl, naphthyl, phenanthryl and anthracenyl; the x bonds and the y bonds are attached to adjacent carbon atoms on the ring structures; each R, when present as a substituent, is independently selected from alkyl, alkoxy, aryloxy, aryl, aralkyl, alkanyl, alkoxyalkyl, cycloaliphatic, halogen (except Cl, Br or I in the ortho position), alkanoyl, alkanoyloxy, alkoxycarbonyl, or formyl; n is a whole number in the range of 0-4 where Ar is phenyl; 0-6 where Ar is naphthyl; and 0-8 where Ar is phenanthryl or anthracenyl; each R1 and R2 is independently selected from alkyl, aryl, aralkyl, alkaryl or cycloaliphatic; each R3 and R4 is independently selected from hydrogen and the R1 substituents; each of the above alkyl groups or moieties is straight or branched chain of 1-20 carbons, preferably 1-8 carbons; each aryl group contains 6-10 ring carbons; each cycloaliphatic group contains from 4-8 ring carbons; and each Y is independently selected from the elements N, P, As, Sb and Bi. Also disclosed are novel ligands wherein one or both of the Ar moieties are phenyl rings.

WO2005/042458 A2 relates to to a method for the continuous production of aldehydes comprising between 5 and 21 carbon atoms, by the isomerising hydroformylation in a homogenous phase of olefin compositions comprising between 4 and 20 carbon atoms and containing alpha-olefins and olefins with internal double bonds, by means of a synthesis gas, in the presence of a homogeneous rhodium catalyst that is complexed with an organophosphorus ligand containing oxygen atoms and/or nitrogen atoms and a free ligand. Said production is carried out at high temperature and high pressure in a multistage reaction system consisting of at least two reaction zones. According to said method, the olefin composition is first reacted in a first reaction zone or a group of several first reaction zones at a total pressure of between 10 and 40 bar, using a synthesis gas with a CO/H2 molar ratio of between 4:1 and 1:2 until a 40 to 95 % conversion of the alpha-olefins is obtained. The hydroformylation product from the first reaction zone or group of several first reaction zones is then reacted in a subsequent reaction zone or group of several reaction zones at a total pressure of between 5 and 30 bar, using a synthesis gas with a CO/H2 molar ratio of between 1:4 and 1:1000. The total pressure in the subsequent reaction zone or zones is respectively 1 to (G1-Gf) bar lower than that of the preceding reaction zone, whereby G1 represents the total pressure in the respective preceding reaction zone and Gf represents the total pressure in the respective reaction zone that succeeds said first reaction zone or zones, with the proviso that the difference between G1 and Gf is greater than 1 bar and the partial CO pressure in the subsequent reaction zone or zones is respectively lower than that of the preceding reaction zone.

US 2007/219399 A1 relates to a hydroformylation process which comprises reacting an olefin with CO and H2 in the presence of a hydroformylation catalyst in a liquid that has been volumetrically expanded with a compressed gas, such as supercritical carbon dioxide.

US 5 675 041 relates to a process for production of C3 to C6 aldehydes by hydro-formylating a mixture containing: (a) C2 to C5 olefins and mixtures thereof, and (b) (i) C2 to C5 alkynes and mixtures thereof or (ii) C3 to C5 cumulated dienes and mixtures thereof or (iii) mixtures of (i) and (ii), with CO, H2 and a solution of a rhodium complex catalyst produced by complexing Rh and an organophosphorus compound at a concentration of Rh in solution from 1 to 1000 ppm by weight. Alternatively, the solution of rhodium complex catalyst can have a P/Rh atom ratio of at least 30. Alternatively, the solution of rhodium complex catalyst can have a P/Rh atom ratio greater than the value RL defined by the formula:

$$R_L = R_B + \frac{(30 - R_B) \cdot 10^{(pKa_{TPP} - pKa_L)}}{e^{(\Delta S_B/R)}}$$

in which $R_B$ is the P/Rh ratio sufficient for a catalytically active Rh complex, $pKa_{TPP}$ is the pKa value for triphenylphosphine, $pKa_L$ is the pKa value for the triorganophosphorus compound, R is the gas constant, and $\Delta S_B$ is 35(N-1) cal/mole/°K., N is the number of P-Rh attachments per ligand molecule. The process has utility for the hydroformylation of streams that contain olefins and alkynes.

US 6 049 011 relates to a continuous process for the hydroformylation of a dilute ethylene feedstream, said process comprising: contacting a dilute ethylene feedstream containing from 27.5 to 75 percent by weight of ethylene, based on the total hydrocarbon content, and having a total olefin content of at most 80 % by weight, based on the total hydrocarbon content, with synthesis gas under hydroformylation conditions in the presence of a rhodium-containing catalyst, wherein said feedstream remains untreated for removal of a compound selected from the group consisting of acetylene, ethane, propane, propene and alkadienes; and recovering a hydroformylation product.

## BRIEF SUMMARY OF THE INVENTION

An embodiment according to the present invention concerns a process for the conversion of olefins into aldehydes as defined in claim 1.

## DETAILED DESCRIPTION

The present invention concerns certain catalyst compositions which can tolerate acetylene impurities in the olefin feed stream. By careful choice of the phosphorus ligand, a rhodium catalyst can be prepared that is unaffected by acetylene levels up to 1000 parts per million in the olefin feed and are still operational at acetylene levels of 2000, 5000, 7500 or even as high as 10000 parts per million. These catalyst compositions are based on electron poor trivalent phosphorus ligands and some specific bidentate ligands.

The present invention presents a catalyst system that can make propionaldehyde from ethylene without sensitivity to acetylene. The value of this catalyst is that it can ride through upsets in the purity of the ethylene supply without having to take extraordinary measures to maintain reactor productivity.

The catalysts of the present invention allow for the preparation of aldehydes from olefin streams that intermittently contain alkyne impurities such as acetylene. The present invention also allows for the preparation of aldehydes from lower quality olefin streams without the requirement of an extra processing step to remove the alkyne impurities.

In the broadest description, this invention is a catalyst and a process for the conversion of ethylene that contains varying amounts of acetylene into propionaldehyde wherein the composition of the catalyst is a rhodium compound in combination with a monodentate triorganophosphorus ligand and a suitable solvent, and the process and the catalyst are unaffected by addition of acetylene to the feed stream at levels up to 1000 ppm.

As used herein, the phrase "substantially free" does not preclude a slight reduction in catalyst conversion activity, and in this regard, a reduction of less than about 10%, 5.0% or even 1.0% conversion activity over a period of at least about 3 hours, at least about 4 hours, or even at least about 5 hours is considered to be a process wherein conversion activity of the catalyst is not reduced.

Rhodium compounds that may be used as a source of rhodium for the active catalyst include rhodium(II) or rhodium(III) salts of carboxylic acids, examples of which include di-rhodium tetraacetate dihydrate, rhodium(II) acetate, rhodium(II) isobutyrate, rhodium(II) 2-ethylhexanoate, rhodium(II) benzoate and rhodium(II) octanoate. Also, rhodium carbonyl species such as $Rh_4(CO)_{12}$, $Rh_6(CO)_{16}$ and rhodium(I) acetylacetonate dicarbonyl may be suitable rhodium feeds. Additionally, rhodium organophosphine complexes such as tris(triphenylphosphine) rhodium carbonyl hydride

may be used when the phosphine moieties of the complex fed are easily displaced by the ligands of the present invention. Less desirable rhodium sources are rhodium salts of strong mineral acids such as chlorides, bromides, nitrates, sulfates, phosphates and the like.

The concentration of the rhodium and ligand in the hydroformylation solvent or reaction mixture can vary. As mentioned hereinabove, a gram mole ligand:gram atom rhodium ratio of at least 1:1 normally is maintained in the reaction mixture. The absolute concentration of rhodium in the reaction mixture or solution may vary from 1 mg/liter up to 5000 mg/liter or more. When the process is operated within the practical conditions of this invention, the concentration of rhodium in the reaction solution normally is in the range of about 10 to 300 mg/liter.

We have found that the fluorophosphite ester compounds having the formula

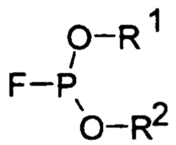

(I)

function as effective ligands when used in combination with transition metals to form catalyst systems for the processes described hereinabove. The hydrocarbyl groups represented by $R^1$ and $R^2$ may be the same or different, separate or combined, and are selected from unsubstituted and substituted alkyl, cycloalkyl and aryl groups containing a total of up to about 40 carbon atoms. The total carbon content of substituents $R^1$ and $R^2$ preferably is in the range of about 2 to 35 carbon atoms. Examples of the alkyl groups which $R^1$ and/or $R^2$ separately or individually can represent include ethyl, butyl, pentyl, hexyl, 2-ethylhexyl, octyl, decyl, dodecyl, octadecyl and various isomers thereof. The alkyl groups may be substituted, for example, with up to two substituents such as alkoxy, cycloalkoxy, formyl, alkanoyl, cycloalkyl, aryl, aryloxy, aroyl, carboxyl, carboxylate salts, alkoxycarbonyl, alkanoyloxy, cyano, sulfonic acid, sulfonate salts and the like. Cyclopentyl, cyclohexyl and cycloheptyl are examples of the cycloalkyl groups $R^1$ and/or $R^2$ individually can represent. The cycloalkyl groups may be substituted with alkyl or any of the substituents described with respect to the possible substituted alkyl groups. The alkyl and cycloalkyl groups which $R^1$ and/or $R^2$ individually can represent preferably are alkyl of up to about 8 carbon atoms, benzyl, cyclopentyl, cyclohexyl or cycloheptyl.

Examples of the aryl groups which $R^1$ and/or $R^2$ individually can represent include carbocyclic aryl such as phenyl, naphthyl, anthracenyl and substituted derivatives thereof. Examples of the carbocyclic aryl groups which $R^1$ and/or $R^2$ individually can represent the radicals having the formulas

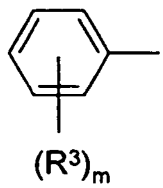

(II)

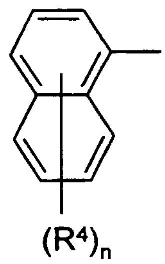

(III)

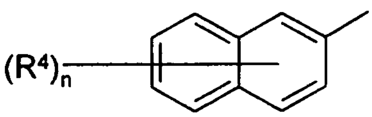

(IV)

wherein $R^3$ and $R^4$ may represent one or more substituents independently selected from alkyl, alkoxy, halogen, cycloalkoxy, formyl, alkanoyl, cycloalkyl, aryl, aryloxy, aroyl, carboxyl, carboxylate salts, alkoxy-carbonyl, alkanoyloxy, cyano, sulfonic acid, sulfonate salts and the like. The alkyl moiety of the aforesaid alkyl, alkoxy, alkanoyl, alkoxycarbonyl and alkanoyloxy groups typically contains up to about 8 carbon atoms. Although it is possible for m to represent 0 to 5

and for n to represent 0 to 7, the value of each of m and n usually will not exceed 2. $R^3$ and $R^4$ preferably represent lower alkyl groups, i.e., straight-chain and branched-chain alkyl of up to about 4 carbon atoms, and m and n each represent 0, 1 or 2.

Alternatively, $R^1$ and $R^2$ in combination or collectively may represent a divalent hydrocarbylene group containing up to about 40 carbon atoms, preferably from about 12 to 36 carbon atoms. Examples of such divalent groups include alkylene of about 2 to 12 carbon atoms, cyclohexylene and arylene. Specific examples of the alkylene and cycloalkylene groups include ethylene, trimethylene, 1,3-butanediyl, 2,2-dimethyl-1,3-propanediyl, 1,1,2-triphenylethanediyl, 2,2,4-trimethyl-1,3-pentanediyl, 1,2-cyclohexylene, and the like. Examples of the arylene groups which $R^1$ and $R^2$ collectively may represent are given herein below as formulas (V), (VI) and (VII).

The divalent groups that $R^1$ and $R^2$ collectively may represent include radicals having the formula

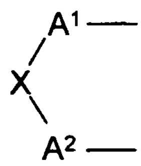

wherein each of $A^1$ and $A^2$ is an arylene radical, e.g., a divalent, carbocyclic aromatic group containing 6 to 10 ring carbon atoms, wherein each ester oxygen atom of fluorophosphite (I) is bonded to a ring carbon atom of $A^1$ and $A^2$;

X is (i) a chemical bond directly between ring carbon atoms of $A^1$ and $A^2$ ; or (ii) an oxygen atom, a group having the formula $--(CH_2)_y--$ wherein y is 2 to 4 or a group having the formula

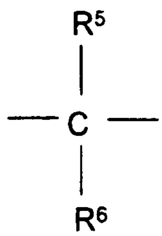

wherein $R^5$ is hydrogen, alkyl or aryl, e.g., the aryl groups illustrated by formulas (II), (III) and (IV), and $R^6$ is hydrogen or alkyl. The total carbon content of the group $--C(R^5)(R^6)--$ normally will not exceed 20 and, preferably, is in the range of 1 to 8 carbon atoms. Normally, when $R^1$ and $R^2$ collectively represent a divalent hydrocarbylene group, the phosphite ester oxygen atoms, i.e. the oxygen atoms depicted in formula (I), are separated by a chain of atoms containing at least 3 carbon atoms.

Examples of the arylene groups represented by each of $A^1$ and $A^2$ include the divalent radicals having the formulas:

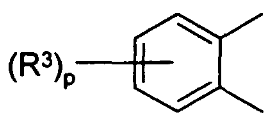

(V)

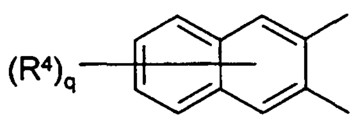

(VI)

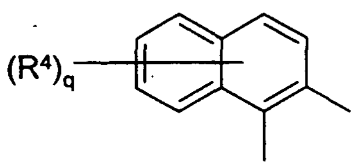

(VII)

wherein $R^3$ and $R^4$ may represent one or more substituents independently selected from alkyl, alkoxy, halogen, cycloalkoxy, formyl, alkanoyl, cycloalkyl, aryl, aryloxy, aroyl, carboxyl, carboxylate salts, alkoxy-carbonyl, alkanoyloxy, cyano, sulfonic acid, sulfonate salts and the like. The alkyl moiety of such alkyl, alkoxy, alkanoyl, alkoxycarbonyl and alkanoyloxy groups typically contains up to about 8 carbon atoms. Although it is possible for p to represent 0 to 4 and for q to represent 0 to 6, the value of each of p and q usually will not exceed 2. $R^3$ and $R^4$ preferably represent lower alkyl groups, i.e., straight-chain and branched-chain alkyl of up to about 4 carbon atoms, and p and q each represent 0, 1 or 2.

Examples of fluorophosphite esters which are useful in the present invention include those which exhibit the best stability, are those wherein the fluorophosphite ester oxygen atoms are bonded directly to a ring carbon atom of a carbocyclic, aromatic group, e.g., an aryl or arylene group represented by any of formulas (II) through (VII). When $R^1$ and $R^2$ individually each represents an aryl radical, e.g., a phenyl group, in one embodiment, 1 or both of the ring carbon atoms that are in a position ortho to the ring carbon atoms bonded to the fluorophosphite ester oxygen atom are substituted with an alkyl group, especially a branched chain alkyl group such as isopropyl, tert-butyl, tert-octyl and the like. Similarly, when $R^1$ and $R^2$ collectively represent a radical having the formula

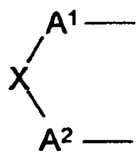

the ring carbon atoms of arylene radicals $A^1$ and $A^2$ that are in a position ortho to the ring carbon atoms bonded to the fluorophosphite ester oxygen atom are substituted with an alkyl group, preferably a branched chain alkyl group such as isopropyl, tert-butyl, tert-octyl and the like. The used in the process according to the invention fluorophosphite esters have the general formula

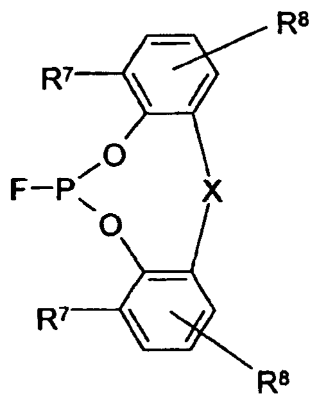

wherein each $R^7$ is alkyl of 3 to 8 carbon atoms; each $R^8$ is hydrogen, alkyl of 1 to 8 carbon atoms or alkoxy of 1 to 8 carbon atoms; and X is (i) a chemical bond directly between ring carbon atoms of each phenylene group to which X is bonded; or (ii) a group having the formula

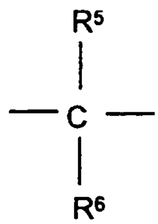

wherein each of $R^5$ and $R^6$ is hydrogen or alkyl of 1 to 8 carbon atoms.

The fluorophosphite esters of formula (I) may be prepared by published procedures or by techniques analogous thereto. See, for example, the procedures described by Riesel et al., J. Z. Anorg. Allg. Chem., 603, 145 (1991), Tullock et al., J. Org. Chem., 25, 2016 (1960), White et al., J. Am. Chem. Soc., 92, 7125 (1970) and Meyer et al., Z. Naturforsch, Bi. Chem. Sci., 48, 659 (1993) and in U.S. Pat. No. 4,912,155.

We have also found that certain bidentate ligands are also useful for the hydroformylation of ethylene containing varying amounts of acetylene. The ligands that have been found to be useful include BISBI and BIPHEPHOS, which do not belong to the invention.

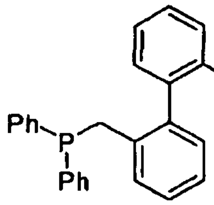

**BISBI**

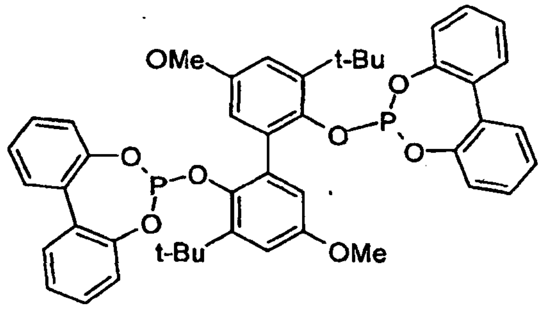

**BIPHEPHOS**

The ratio of gram moles phosphorus ligand to gram atoms transition metal can vary over a wide range, e.g., gram mole phosphorus:gram atom transition metal ratios of about 1:1 to 1000:1. For the rhodium-containing catalyst systems based on phosphite and fluorophosphite ligands, the gram mole phosphorus:gram atom rhodium ratio can be in the range of about 100:1 up to about 750:1, about 200:1 to about 400:1 or even about 250:1 to 300:1.

Different classes of phosphorus ligands such phosphines will exhibit very different behaviors and will require different phosphorus to rhodium molar ratios. Bidentate ligands which chelate strongly to the rhodium atom can be run at much lower ligand to rhodium molar ratios. For example, BISBI and BIPHEPHOS are bidentate phosphine and phosphite ligands respectfully and both have been shown to form stable, acetylene tolerant catalyst systems at ligand to rhodium molar ratios of about 1:1 to about 90:1 or from about 30:1 to about 80:1.

The hydroformylation reaction solvent may be selected from a wide variety of compounds, mixture of compounds, or materials that are liquid at the pressure at which the process is being operated. Such compounds and materials include various alkanes, cycloalkanes, alkenes, cycloalkenes, carbocyclic aromatic compounds, alcohols, esters, ketones, acetals, ethers and water. Specific examples of such solvents include alkane and cycloalkanes such as dodecane, decalin, octane, iso-octane mixtures, cyclohexane, cyclooctane, cyclododecane, methylcyclohexane; aromatic hydrocarbons such as benzene, toluene, xylene isomers, tetralin, cumene, alkyl-substituted aromatic compounds such as the isomers of diisopropylbenzene, triisopropylbenzene and tert-butylbenzene; crude hydrocarbon mixtures such as naphtha, mineral oils and kerosene; high-boiling esters such as 2,2,4-trimethyl-1,3-pentanediol diisobutyrate. The aldehyde product of the hydroformylation process also may be used. In one embodiment, a useful solvent is the higher boiling by-products that are naturally formed during the process of the hydroformylation reaction and the subsequent steps, e.g., distillations, that are required for aldehyde product isolation. The main criteria for the solvent are that it dissolves the catalyst and olefin substrate and that it does not act as a poison to the catalyst. In an embodiment, useful solvents for the production of volatile aldehydes, e.g., propionaldehyde and the butyraldehydes, are those that are sufficiently high boiling to remain for the most part in a gas sparged reactor. Solvents and solvent combinations that can be used in the production of less volatile and non-volatile aldehyde products include 1-methyl-2-pyrrolidinone, dimethylformamide, perfluorinated solvents such as perfluoro-kerosene, sulfolane, water, and high boiling hydrocarbon liquids as well as combinations of these solvents.

The reaction conditions used are not critical for the operation of the process and conventional hydroformylation conditions normally are used. The process requires that an olefin is contacted with hydrogen and carbon monoxide in the presence of the novel catalyst system described hereinabove. The process may be carried out at temperatures in the range of about 20° to 200° C., or reaction temperatures are from 50° to 135° C or reaction temperatures ranging from 75° to 125° C.

The hydrogen:carbon monoxide molar ratio in the reactor likewise may vary considerably ranging from 10:1 to 1:10 and the sum of the absolute partial pressures of hydrogen and carbon monoxide may range from 0.3 to 36 bars absolute. The partial pressures of the hydrogen and carbon monoxide in the feed is selected according to the linear:branched isomer ratio desired. Generally, the partial pressure of hydrogen in the reactor can be maintained within the range of about 1.4 to about13.8 bars absolute (about 20 to about 200 psia). The partial pressure of carbon monoxide in the reactor can be maintained within the range of about 1.4 to about 13.8 bars absolute (about 20 to about 200 psia) or from about 4 to about 9 bars absolute and may be varied independently of the hydrogen partial pressure. The molar ratio of hydrogen to carbon monoxide can be varied widely within these partial pressure ranges for the hydrogen and carbon monoxide. The ratios of the hydrogen to carbon monoxide and the partial pressure of each in the feed gas stream (often referred to as synthesis gas or syn gas) can be readily changed by the addition of either hydrogen or carbon

monoxide to the synthesis gas stream.

The catalysts of the present invention can successfully hydroformylate acetylene contaminated ethylene to produce propionaldehyde without loss of activity. Acetylene levels of contamination up to 1000 ppm do not cause any loss of activity while levels of 10,000 ppm will cause some loss of activity.

Any of the known hydroformylation reactor designs or configurations may be used in carrying out the process provided by the present invention. Thus, a gas-sparged, vapor take-off reactor design as disclosed in the examples set forth herein may be used. In this mode of operation, the catalyst which is dissolved in a high boiling organic solvent under pressure does not leave the reaction zone with the aldehyde product which is taken overhead by the unreacted gases. The overhead gases then are chilled in a vapor/liquid separator to liquefy the aldehyde product and the gases can be recycled to the reactor. The liquid product is let down to atmospheric pressure for separation and purification by conventional techniques. The process also may be practiced in a batchwise manner by contacting the olefin, hydrogen and carbon monoxide with the present catalyst in an autoclave.

A reactor design where catalyst and feedstock are pumped into a reactor and allowed to overflow with product aldehyde, i.e. liquid overflow reactor design, is also suitable. For example, high boiling aldehydes products such as nonyl aldehydes may be prepared in a continuous manner with the eldehyde product being removed from the reactor zone as a liquid in combination with the catalyst. The aldehyde product may be separated from the catalyst by conventional means such as by distillation or extraction and the catalyst then recycled back to the reactor. Water soluble aldehyde products, such as hydroxyl Butyraldehyde products obtained by the hydroformylation of allyl alcohol, can be separated from the catalyst by extraction techniques. A trickle-bed reactor design also is suitable for this process. It will be apparent to those skilled in the art that other reactor schemes may be used with this invention.

No special or unusual techniques are required for preparing the catalyst systems and solutions of the present invention, although, to obtain a catalyst of high activity, all manipulations of the rhodium and the fluorophosphite ligand components should be carried out under an inert atmosphere, e.g., nitrogen, argon and the like. The desired quantities of a suitable rhodium compound and ligand are charged to the reactor in a suitable solvent. The sequence in which the various catalyst components or reactants are charged to the reactor is not critical.

The hydroformylation reaction of ethylene to produce propionaldehyde is practiced on large industrial scale. The propionaldehyde serves as a feedstock for other useful chemicals including propionic acid and propanol.

The work described herein was conducted in a continuous operation bench unit as described in U. S. Patent 5,840,647. The hydroformylation of ethylene to produce propionaldehyde was carried out in a vapor take-off reactor consisting of a vertically arranged stainless steel pipe having a 2.5 cm inside diameter and a length of 1.2 meters. The reactor was encased in an external jacket that was connected to a hot oil machine. The reactor had a filter element welded into the side down near the bottom of the reactor for the inlet of gaseous reactants. The reactor contained a thermowell arranged axially with the reactor in its center for accurate measurement of the temperature of the hydroformylation reaction mixture. The bottom of the reactor had a high pressure tubing connection that was connected to a cross. One of the connections to the cross permitted the addition of non-gaseous reactants such as octene-1 or make-up solvent, another led to the high-pressure connection of a differential pressure (D/P) cell that was used to measure catalyst level in the reactor and the bottom connection was used for draining the catalyst solution at the end of the run.

In the hydroformylation of ethylene in a vapor take-off mode of operation, the hydroformylation reaction mixture or solution containing the catalyst was sparged under pressure with the incoming reactants of ethylene, hydrogen, and carbon monoxide as well as any inert feed such as nitrogen. As propionaldehyde was formed in the catalyst solution, it and unreacted reactant gases were removed as a vapor from the top of the reactor by a side-port. The vapor removed was chilled in a high-pressure separator where the propionaldehyde product was condensed. The uncondensed gases were let down to atmospheric pressure via the pressure control valve. These gases passed through a series of dry-ice traps where any other aldehyde product was collected. The product from the high-pressure separator was combined with that of the traps, and was subsequently weighed and analyzed by standard gas/liquid phase chromatography (GLC) techniques for the net weight and propionaldehyde product.

The gaseous feeds to the reactor were fed to the reactor via twin cylinder manifolds and high-pressure regulators. The hydrogen passed through a mass flow controller and then through a commercially available DEOXO® (available from Engelhard Inc.) catalyst bed to remove any oxygen contamination. The carbon monoxide passed through an iron carbonyl removal bed (as disclosed in U.S. Patent 4,608,239), a similar DEOXO® bed heated to 125°C, and then a mass flow controller. Nitrogen could be added to the feed mixture as an inert gas. Nitrogen, when added, was metered in and then mixed with the hydrogen feed prior to the hydrogen DEOXO® bed. Ethylene was fed to the reactor from cylinders and was controlled using a mass flow meter. All gases feeds were passed through a preheater prior to entering the reactor.

This invention can be further illustrated by the following examples of preferred embodiments thereof, although it will be understood that these examples are included merely for purposes of illustration and are not intended to limit the scope of the invention.

## Examples

A bench unit catalyst was prepared from rhodium 2-ethylhexanoate, dioctylphthalate solvent, and the phosphorus ligand as noted in the table. The catalyst was charged to the continuous bench unit reactor, heated to temperature and fed the reactants. The unit was operated for three hours using a pure ethylene feed as the olefin feed. The catalyst activity in the third hour was taken as a base point for further comparisons and then the olefin feed was switched from pure ethylene to ethylene containing 1000 ppm acetylene. The catalyst activity for the ensuing hours was observed and recorded in Table I.

The data in Table I shows that phosphine based catalysts are strongly negatively affected by acetylene when compared with the fluorophosphite based catalysts of the present invention. In particular, the more basic ligands such as tricyclohexylphosphine and tribenzylphosphine are more strongly affected. The abbreviations used in the table are: TBzP is tribenzylphosphine; TPP is triphenylphosphine; TCHP is tricyclohexylphosphine; and Ethanox 398™ is 2,2'-Ethylidene-bis-(4,6-di-*tert*-butylphenyl)fluorophosphite.

Lines 3 and 4 in Table I illustrate the value of and the stabilizing effect of increasing the ligand to rhodium molar ratio.

Table I. Hydroformylation of Ethylene Containing 1000 Parts Per Million Acetylene

| Ligand | Millimole of ligand used | Rhodium milligrams | Ligand/Rh mole ratio | Catalyst Activity, grams of propionaldehyde (% of hour 3 activity retained) | | | Reference |
|---|---|---|---|---|---|---|---|
| | | | | Hour 3 | Hour 4 | Hour 5 | |
| Ethanox 398™ | 8.0 | 3.0 | 275 | 83.1 | 84.3 (101%) | 84.1 (101%) | X-29055-022 |
| Ethanox 398™ | 8.0 | 3.0 | 275 | 60.6 | 57.1 (94%) | 55.2 (91%) | X-29055-0520 |
| 2,2'-methylidene-bis-(6-di-*tert*-butyl-4-dimethylphenyl) fluorophosphite | 8.0 | 3.0 | 274 | 67.0 | 70 (104%) | 67.4 (101%) | X-29055-092 |
| 2.2'-methylidene-bis-(6-di-*tert*-butyl-4-dimethylphenyl) fluorophosphite | 1.2 | 3.0 | 41 | 126.3 | 106.1 | 31.4 | X-29055-090 |
| TBzP, | 5.3 | 10.0 | 55 | 44.6 | 36.5 (82%) | 19.4 (43%) | X-29055-024 |
| TCHP | 5.3 | 10.0 | 55 | 25.0 | 7.6 (30%) | 2.4 (10%) | X-29055-028 |
| TPP | 17.0 | 5.0 | 350 | 96.0 | 32.8 (34%) | 7.2 (8%) | X-29055-108 |
| TPP | 29.1 | 5.0 | 599 | 162.5 | 48.5 (30%) | 19.4 (12%) | X-29055-114 |
| TPP | 40.0 | 6.0 | 687 | 100.8 | 90.3 (90%) | 60.2 (60%) | X-29055-026 |
| TCHP & TBP | 2.7 mmole each | 20 | 28 | 20.7 | 13.4 (65%) | 6.2 (30%) | X-29055-038 |
| All runs were made at 105°C, 18.9 barg; (260 psig) with 1:1 syn gas (13.2 bara; 192 psia), $N_2$ (2 bara; 29 psia), $C_2H_4$ (3.7 bara 54 psia) with a total gas flow of 9.6 liters/minute in 190 milliliters of DOP solvent | | | | | | | |
| Ligands TBzP, TCHP, TPP, TCHP & TBP are for comparison. | | | | | | | |

The table shows that all of the ligands other than the fluorophosphites (Ethanox 398™ and 2,2'-methylidene-bis-(6-di-*tert*-butyl-4-dimethylphenyl) fluorophosphite) show some degree of catalyst activity loss with the introduction of acetylene. The severity of the catalyst activity loss is a function of the ligand. The strongly basic ligand, TCHP, shows the most significant loss of activity. The triphenylphosphine based catalyst systems shows less of a loss of activity at higher ligand loadings as taught by Fell and Beutler and also in U. S. Patent 5,675,041.

We have also found that certain bidentate ligands can be used for the hydroformylation of ethylene and that acetylene at 1000 ppm has no effect on these bidentate ligands. The entries in table II are two bidentate ligands. BISBI is the bidentate phosphine ligand 1,1'-(2,2'-bisdiphneylphosphinomethyl) biphenyl. The BIPHEPHOS ligand is a bidentate bis phosphite ligand, 2,2'-Bis[(1,1'-biphenyl-2,2'-diyl)phosphite]-3,3'-di-tert-butyl-5,5'-dimethoxy-1,1'-biphenyl; CAS registry number 121627-17-6. Neither of these bidentate ligands was strongly affected by the acetylene contaminant at 1000 ppm. The experimental work of Table I above was repeated using an ethylene feed that was spiked to 1,000 ppm of acetylene. The reactions were monitored for two half hour intervals after the introduction of the acetylene containing feed. The data from these runs is presented in Table II (Comparative Examples).

Table II - Acetylene Tolerance of Bidentate Ligand based Catalysts

| Ligand | Millimole of ligand used | Rhodium milligrams | Catalyst Activity, grams of propionaldehyde (% of hour 3 activity retained) | | | Reference |
|---|---|---|---|---|---|---|
| | | | Hour 3 | Hour 3.5 | Hour 4.0 | |
| BISBI | 3.0 | 4.0 | 75.3 | 71.5 (95%) | 75.3 (100%) | X-29055-036 |
| BIPHEPH OS | 2.5 | 3.0 | 48.4 | 52.2 (107%) | 51.2 (105%) | X-29055-040 |
| All runs were made at 105°C (18.9 barg; 260 psig) with 1:1 syn gas (13.2 bara;192 psia), $N_2$ (2 bara; 29 psia), $C_2H_4$ (3.7 bara; 54 psia) with a total gas flow of 9.6 liters/minute in 190 milliliters of DOP solvent | | | | | | |

The experimental work of Table I above was repeated using an ethylene feed that was spiked to 10,000 ppm of acetylene. The reactions were monitored for two half hour intervals after the introduction of the acetylene containing feed. The acetylene at 10,000 ppm was sufficient to have a negative effect on all catalysts including the Ethanox 398™ based catalyst. However, the Ethanox 398™ based catalyst shows the greatest retention of activity after two hours of operation with the 10,000 ppm acetylene feed. The results of these experiments are presented in the table below, wherein ligand TPP is for comparison.

Table II. Hydroformylation of Ethylene Containing 10,000 Parts Per Million of Acetylene

| Ligand, Reaction Temperature | Millimole of ligand used | Ligand to Rh Molar Ratio | Rhodium milligrams | Catalyst Activity, grams of propionaldehyde (% of hour 3 activity retained) | | | Reference |
|---|---|---|---|---|---|---|---|
| | | | | Hour 3 | Hour 3½ | Hour 4 | |
| TPP, 105C | 17.0 | 87 | 5 | 94.8 | 32.9 (69%) | 7.0 (15%) | X-29055-108 |
| TPP, 105C | 29.1 | 150 | 5 | 78.7 | 28.8 (73%) | 9.1 (23%) | X-29055-112 |
| TPP, 115C | 29.1 | 150 | 5 | 162.5 | 48.5 (60%) | 19.4 (24%) | X-29055-114 |
| Ethanox 398™, 120C | 8.0 | 412 | 2 | 81.5 | 22.0 (54%) | 15.7 (39%) | X-29055-116 |
| Ethanox 398™, 110C | 8.0 | 274 | 3 | 85.4 | 79.0 (93%) | 38.2 (45%) | X-29055-098 |
| All runs were made at (18.9 barg; 260 psig) with 1:1 syn gas (13.2 bara; 192 psia), $N_2$ (2 bara; 29 psia), $C_2H_4$ (3.7 bara, 54 psia) with a total gas flow of 9.6 liters/minute in 190 milliliters of DOP solvent | | | | | | | |

## Claims

1. A process for the conversion of olefins into aldehydes, comprising contacting an olefin stream with a catalyst in a solvent to form at least one aldehyde,
wherein
the catalyst is a rhodium compound in combination with a ligand,
the ligand to rhodium molar ratio is from 1 : 1 to 1000 : 1, and
said olefin stream contains acetylene, wherein said acetylene does not reduce the conversion activity of the catalyst, which means that the conversion activity of the catalyst is reduced less than 1.0 %, 5.0 % or 10 % over a period of at least 3 hours, at least 4 hours or at least 5 hours;
the olefin is ethylene and the aldehyde is propionaldehyde;
the concentration of rhodium in the reaction solution is in the range of from 10 to 300 mg/liter;
the ligand is a fluorophosphite ester having the general formula

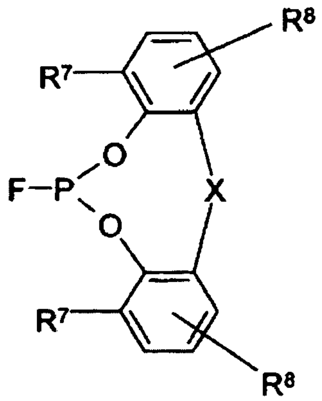

wherein each $R^7$ is alkyl of 3 to 8 carbon atoms; each $R^8$ is hydrogen, alkyl of 1 to 8 carbon atoms or alkoxy of 1 to 8 carbon atoms; and X is (i) a chemical bond directly between ring carbon atoms of each phenylene group to which X is bonded; or (ii) a group having the formula

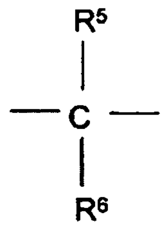

wherein each of $R^5$ and $R^6$ is hydrogen or alkyl of 1 to 8 carbon atoms;
the gram mole phosphorus : gram atom rhodium ratio is in the range of from 100 : 1 up to 750 : 1 , or from 200 : 1 to 400 : 1 or from 250 : 1 to 300 : 1;
the process requires that ethylene is contacted with hydrogen and carbon monoxide in the presence of the catalyst;
the process is carried out at temperatures in the range of 20 °C to 200 °C, or reaction temperatures are from 50 °C to 135 °C, or reaction temperatures ranging from 75 °C to 125 °C;
the hydrogen : carbon monoxide molar ratio ranges from 10 : 1 to 1 : 10 and the sum of the absolute partial pressures of hydrogen and carbon monoxide is from 0.3 to 36 bars absolute, wherein the partial pressure of hydrogen is maintained in the range of from 1.4 to 13.8 bars absolute, the partial pressure of carbon monoxide is maintained within the range of from 1.4 to 13.8 bars absolute or from 4 to 9 bars absolute and may be varied independently of the hydrogen partial pressure; and
wherein the upper limit of the acetylene level is 10,000 ppm.

2. The process of claim 1, wherein the ligand is 2,2'-ethylidene-bis-(4,6-di-tert.-butylphenyl)fluorophosphite or 2,2'-methylidene-bis-(6-di-tert.-butyl-4-dimethylphenyl)fluorophosphite.

**Patentansprüche**

1. Verfahren zur Umwandlung von Olefinen in Aldehyde, umfassend das Kontaktieren eines Olefinstroms mit einem Katalysator in einem Lösungsmittel, um mindestens einen Aldehyd zu bilden,
wobei
der Katalysator eine Rhodiumverbindung in Kombination mit einem Liganden ist, das molare Verhältnis von Ligand zu Rhodium 1 : 1 bis 1.000 : 1 beträgt, und besagter Olefinstrom Acetylen enthält, wobei besagtes Acetylen die Umwandlungsaktivität des Katalysators nicht erniedrigt, was bedeutet, dass die Umwandlungsaktivität des Katalysators um weniger als 1,0 %, 5,0 % oder 10 % über eine Zeitdauer von mindestens 3 Stunden, mindestens 4 Stunden oder mindestens 5 Stunden reduziert wird;
das Olefin Ethylen und der Aldehyd Propionaldehyd ist;
die Konzentration an Rhodium in der Reaktionslösung im Bereich von 10 bis 300 mg/l liegt;
der Ligand ein Fluorphosphitester der allgemeinen Formel

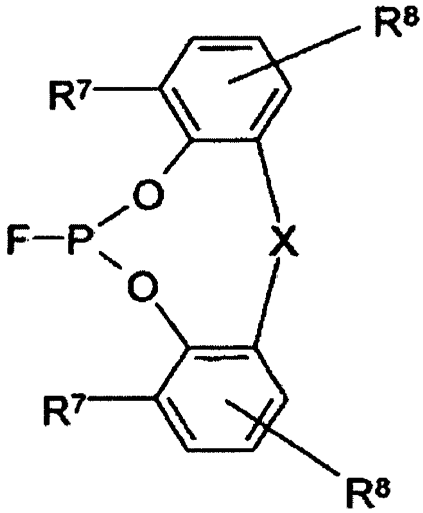

ist, wobei jedes $R^7$ Alkyl mit 3 bis 8 Kohlenstoffatomen ist; jedes $R^8$ Wasserstoff, Alkyl mit 1 bis 8 Kohlenstoffatomen oder Alkoxy mit 1 bis 8 Kohlenstoffatomen ist; und X (i) eine chemische Bindung direkt zwischen Ringkohlenstoffatomen einer jeden Phenylengruppe ist, an welche X gebunden ist; oder (ii) eine Gruppe der Formel

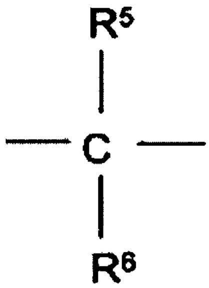

ist, wobei jedes $R^5$ und $R^6$ Wasserstoff oder Alkyl mit 1 bis 8 Kohlenstoffatomen ist; das Verhältnis Grammmole Phosphor: Grammatom Rhodium im Bereich von 100 : 1 bis zu 750 : 1 oder im Bereich von 200 : 1 bis 400 : 1 oder im Bereich von 250 : 1 bis 300 : 1 liegt;
das Verfahren erfordert, dass Ethylen mit Wasserstoff und Kohlenstoffmonoxid in Gegenwart des Katalysators kontaktiert wird;
das Verfahren bei Temperaturen im Bereich von 20 °C bis 200 °C oder bei Reaktionstemperaturen von 50 °C bis 135 °C oder Reaktionstemperaturen im Bereich von 75 °C bis 125 °C durchgeführt wird;
das molare Verhältnis von Wasserstoff : Kohlenmonoxid im Bereich von 10 : 1 bis 1 : 10 liegt und die Summe der absoluten Partialdrucke von Wasserstoff und Kohlenstoffmonoxid 0,3 bis 36 bar absolut beträgt, wobei der Partialdruck von Wasserstoff im Bereich von 1,4 bis 13,8 bar absolut und der Partialdruck von Kohlenstoffmonoxid im Bereich von 1,4 bis 13,8 bar absolut oder von 4 bis 9 bar absolut gehalten wird und unabhängig vom Wasserstoffpartialdruck variieren kann; und
wobei die obere Grenze des Acetylenniveaus 10.000 ppm beträgt.

**2.** Verfahren nach Anspruch 1, wobei der Ligand 2,2'-Ethyliden-bis-(4,6-di-tert.-butylphenyl)fluorphosphit oder 2,2'-Methyliden-bis-(6-di-tert.-butyl-4-dimethylphenyl)fluorphosphit ist.

## Revendications

**1.** Procédé pour la conversion d'oléfines en aldéhydes, comprenant la mise en contact d'un courant d'oléfine avec un catalyseur dans un solvant pour former au moins un aldéhyde,
dans lequel
le catalyseur est un composé de rhodium en combinaison avec un ligand,
le rapport molaire ligand à rhodium est de 1:1 à 1000:1, et ledit courant d'oléfine contient de l'acétylène, dans lequel ledit acétylène ne réduit pas l'activité de conversion du catalyseur, ce qui indique que l'activité de conversion du catalyseur est réduite de moins de 1,0 %, 5,0 % ou 10 % sur une période d'au moins 3 heures, d'au moins 4 heures ou d'au moins 5 heures ;
l'oléfine est l'éthylène et l'aldéhyde est le propionaldéhyde ;
la concentration en rhodium dans la solution réactionnelle se trouve dans l'intervalle de 10 à 300 mg/litre ;
le ligand est un ester de fluorophosphite présentant la formule générale

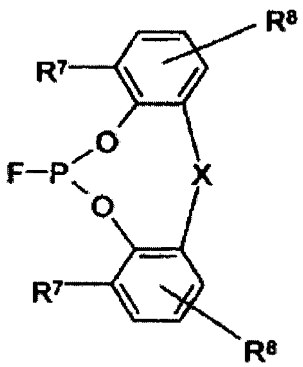

où chaque $R^7$ est un groupe alkyle de 3 à 8 atomes de carbone ; chaque $R^8$ est un atome d'hydrogène, un groupe alkyle de 1 à 8 atomes de carbone ou alcoxy de 1 à 8 atomes de carbone ; et X est (i) une liaison chimique directement entre des atomes de carbone de cycle de chaque groupe phénylène auquel X est lié ; ou (ii) un groupe présentant la formule

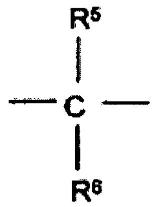

où chacun de $R^5$ et $R^6$ est un atome d'hydrogène ou un groupe alkyle de 1 à 8 atomes de carbone ;
le rapport gramme mole de phosphore : gramme atome de rhodium se trouve dans l'intervalle de 100:1 à 750:1, ou de 200:1 à 400:1 ou de 250:1 à 300:1 ;
le procédé exige que de l'éthylène est mis en contact avec de l'hydrogène et du monoxyde de carbone en présence du catalyseur ;
le procédé est réalisé à des températures dans l'intervalle de 20°C à 200°C, ou les températures de réaction sont de 50°C à 135°C, ou les températures de réaction sont de 75°C à 125°C ;
le rapport molaire hydrogène:monoxyde de carbone est de 10:1 à 1:10 et la somme des pressions partielles absolues de l'hydrogène et du monoxyde de carbone est de 0,3 à 36 bars absolus, dans lequel la pression partielle de l'hydrogène est maintenue dans l'intervalle de 1,4 à 13,8 bars absolus, la pression partielle du monoxyde de carbone

est maintenue dans l'intervalle de 1,4 à 13,8 bars absolus ou de 4 à 9 bars absolus et peut varier indépendamment de la pression partielle d'hydrogène ; et dans lequel la limite supérieure de la teneur en acétylène est de 10 000 ppm.

2. Procédé selon la revendication 1, dans lequel le ligand est le 2,2'-éthylidène-bis-(4,6-di-tert.-butylphényl)fluorophosphite ou le 2,2'-méthylidène-bis-(6-di-tert.-butyl-4-diméthylphényl)fluorophosphite.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8707600 A2
- WO 2005042458 A2
- US 2007219399 A1
- US 5675041 A
- US 6049011 A
- US 4912155 A
- US 5840647 A
- US 4608239 A


### Non-patent literature cited in the description


- **RIESEL et al.** *J. Z. Anorg. Allg. Chem.,* 1991, vol. 603, 145
- **TULLOCK et al.** *J. Org. Chem.,* 1960, vol. 25, 2016
- **WHITE et al.** *J. Am. Chem. Soc.,* 1970, vol. 92, 7125
- **MEYER et al.** *Z. Naturforsch, Bi. Chem. Sci.,* 1993, vol. 48, 659